# EUROPÄISCHE PATENTSCHRIFT

(11) **EP 4 271 427 B1**
(45) Veröffentlichungstag und Bekanntmachung des Hinweises auf die Patenterteilung: **18.09.2024**
(21) Anmeldenummer: 21844723.3
(22) Anmeldetag: 23.12.2021
(51) Int. Cl.: A61L 15/46, A61L 27/30, A61L 27/50, A61L 27/54, A61L 27/56, A61L 15/18

(54) **BESCHICHTUNG UND VERFAHREN ZUM BESCHICHTEN EINES SUBSTRATS**
COATING, AND METHOD FOR COATING A SUBSTRATE
REVÊTEMENT ET PROCÉDÉ DE REVÊTEMENT D'UN SUBSTRAT

(30) Priorität: 29.12.2020 DE 102020135064
(43) Veröffentlichungstag der Anmeldung: 08.11.2023
(73) Patentinhaber: Ara-Coatings GmbH & Co. KG, 48529 Nordhorn (DE); Bio-Gate AG, 90411 Nürnberg (DE)
(72) Erfinder: DOMNICK, Ralph, 48531 Nordhorn (DE); STEINRÜCKE, Peter, 90461 Nürnberg (DE)
(74) Vertreter: Meyer, Rudolf
(86) Internationale Anmeldenummer: PCT/EP2021/087496
(87) Internationale Veröffentlichungsnummer: WO 2022/144299

(56) Entgegenhaltungen:
- DE-A1- 102008 001 014
- US-A1- 2009 035 341

## Beschreibung

Die Erfindung betrifft eine für die Verwendung im Medizintechnik- oder Hygienebereich geeignete Beschichtung. Ferner betrifft die Erfindung ein Verfahren zum Beschichten eines Substrats.

Die DE 10 2010 054 046 B4 offenbart eine für ein Implantat vorgesehene antibakterielle Beschichtung, welche eine Schicht aus Kupfer-Titan-Nitrid enthält. Durch den Kupfergehalt der Beschichtung soll die antibakterielle Wirkung von Kupfer genutzt werden, ohne wesentliche Nachteile hinsichtlich mechanischer Eigenschaften der Beschichtung, insbesondere der Härte, in Kauf nehmen zu müssen. Optional enthält die Beschichtung zusätzlich Zirkon, womit eine besonders hohe Härte erzielbar sein soll. Zusätzlich zu Kupfer enthaltenden Schichten können Verzögerungsschichten vorgesehen sein, die keinen KupferAnteil enthalten. Hiermit soll es möglich sein, die Freisetzungsrate von Kupferionen an die Umgebung zu kontrollieren. Neben Kupfer kann die bekannte Beschichtung mindestens einen der Bestandteile Ti, Nb, Ta, Cr, Mo, W, Si, Al enthalten.

Die EP 2 281 590 A2 beschreibt ein biokorrodierbares Implantat mit einer aktiven Beschichtung. Dieses Implantat weist einen Grundkörper aus einem biokorrodierbaren, metallischen Implantatwerkstoff auf, wobei eine aktive Beschichtung und/oder Kavitätenfüllung wenigstens eine antioxidative Substanz enthält. Bei der antioxidativen Substanz handelt es sich beispielsweise um Squalen oder Gallate.

Ein in der EP 1 790 224 B1 beschriebenes antimikrobielles und nicht zytotoxisches Schichtmaterial umfasst eine Biozid-Schicht, welche Silber, Kupfer und/oder Zink enthält, sowie eine die Biozid-Schicht bedeckende Transportkontrollschicht, deren Dicke und Porosität derart eingestellt sind, dass der biozide Wirkstoff durch die Transportkontrollschicht hindurch in einer antimikrobiellen und nicht zytotoxischen Menge abgegeben wird. Bei einem Grundmaterial der Transportkontrollschicht kann es sich beispielsweise um ein Plasmapolymer, ein Sol-Gel oder einen Lack handeln. In jedem Fall weist die Transportkontrollschicht einen Silizium- und einen Kohlenstoffanteil auf. Zur Herstellung des Schichtmaterials werden in der EP 1 790 224 B1 insbesondere vakuumgestützte Dünnschichtverfahren vorgeschlagen.

Aus der DE 10 2008 001 014 A1 ist ein antimikrobielles und nicht zytotoxisches Schichtmaterial bekannt, welches eine Transportkontrollschicht umfasst, deren Gasdurchlässigkeit für Sauerstoff 50 bis unter 100 (cm³ bar)/(Tag/m²) beträgt. Darüber hinaus ist auch in diesem Fall ein anorganisches Biozid Bestandteil des Schichtmaterials.

Die WO 2007/051519 A2 offenbart eine offenporige biokompatible Oberflächenschicht für ein Implantat. Hierbei sollen Poren der Oberflächenschicht zu einem zusammenhängenden Porennetzwerk verbunden sein. Zwischen der Rohoberfläche des Implantats und der Oberflächenschicht kann eine Zwischenschicht vorgesehen sein, die Titan und/oder Silizium enthält.

Die EP 1 924 300 B1 beschreibt ein Verfahren zum Herstellen eines porösen Überzugs auf einem medizinischen Implantat mit Strukturen im Mikro- oder Nanometerbereich. Im Rahmen dieses Verfahrens werden auf einer Oberfläche temporäre Teilchen abgeschieden, welche in einem späteren Verfahrensschritt wieder entfernt werden, um die gewünschte Porosität des Überzugs zu erhalten. Die erhaltenen Poren können wenigstens teilweise mit Arzneimitteln oder biologisch wirksamen Keramikmaterialien gefüllt werden.

Eine in der WO 2007/051806 A1 beschriebene Mischung für eine Beschichtungsverfahren umfasst eine vernetzbare Flüssigkeit sowie einen Funktionsbestandteil, der unter anderem antimikrobielle Wirkstoffe, Korrosionsschutzinhibitoren und Farbpigmente enthalten kann. Die unter Verwendung der in der WO 2007/051806 A1 beschriebenen Mischung hergestellte Beschichtung soll beispielsweise als Diffusionsbarriere-Beschichtung gegenüber Flüssigkeiten, Gasen und/oder Dämpfen verwendbar sein.

Die WO 2019/121667 A1 offenbart ein antimikrobielles Schichtmaterial, welches eine Schicht mit einem partikulären bioziden Wirkstoff und eine darauf angeordnete Transportkontrollschicht umfasst. Zur Abscheidung der Transportkontrollschicht wird ein After-Glow PE-CVD-Verfahren vorschlagen. Das Schichtmaterial nach der WO 2019/121667 A1 ist insbesondere für die Beschichtung von Medizinprodukten für human- und/oder veterinärmedizinische Anwendungen vorgesehen. Die Transportkontrollschicht kann neben Sauerstoff und Kohlenstoff beispielsweise Silizium oder Titan enthalten. Als biozider Wirkstoff kommt Silber, Kupfer, Zink oder ein organisches Biozid in Betracht.

Aus der DE 10 2012 100 288 A1 ist ein Kunststoffsubstrat mit einer porösen Schicht bekannt, wobei die poröse Schicht zumindest teilweise aus dem Material des Kunststoffsubstrats gebildet ist. Der Volumenanteil der Poren soll in einem ersten Bereich der porösen Schicht größer als in einem zweiten Bereich der porösen Schicht sein. Die Herstellung der porösen Schicht soll mittels eines Plasmaprozesses erfolgen.

Ein Verfahren zum Sintern einer porösen Beschichtung ist beispielsweise in der WO 03/025783 A2 beschrieben. Hierbei ist der Aufbau der porösen Beschichtung auf einem Substrat vorgesehen, welches Öffnungen, insbesondere in Form von Poren, aufweist. Durch geeignete Einstellung der Viskosität einer Paste, aus welcher die Beschichtung gewonnen wird, soll das Auffüllen der im Substrat befindlichen Poren beim Beschichtungsvorgang vermieden werden.

Die WO 2008/040666 A1 offenbart eine transparente poröse SiO₂ Beschichtung für ein transparentes Substratmaterial. Als mögliches Substratmaterial ist Polycarbonat genannt. Zur Herstellung der Beschichtung wird ein Sol-Gel-Verfahren vorgeschlagen.

Der Erfindung liegt die Aufgabe zu Grunde, gegenüber dem genannten Stand der Technik weiterentwickelte, Silizium enthaltende, insbesondere für den Medizintechnik- und Hygienebereich geeignete, Beschichtungen bereitzustellen, welche sich durch in vielfältiger Weise anwendungsspezifisch anpassbare Eigenschaften auszeichnen.

Diese Aufgabe wird erfindungsgemäß gelöst durch eine Beschichtung mit den Merkmalen des Anspruchs 1. Ebenso wird die Aufgabe gelöst durch ein Verfahren zum Beschichten eines Substrats gemäß Anspruch 16. Im Folgenden im Zusammenhang mit dem Beschichtungsverfahren erläuterte Ausgestaltungen und Vorteile der Erfindung gelten sinngemäß auch für die Vorrichtung, das heißt die auf einem beliebigen Substrat befindliche Beschichtung, und umgekehrt.

Die Beschichtung umfasst mindestens zwei verschiedene Teilbereiche, wobei mindestens einer dieser Teilbereiche als poröse, mit Flüssigkeit beladbare, Silizium als Hauptbestandteil enthaltende Schicht ausgebildet und ein weiterer Teilbereich mehrschichtig, nämlich aus einer Biozid-Schicht und einer diese abdeckenden Transportkontrollschicht, aufgebaut ist, und wobei die poröse Schicht einen Siliziumanteil (in Gew.-%) aufweist, der mindestens das 1,4-fache und höchstens das 5-fache des Siliziumanteils der Transportkontrollschicht beträgt.

Die unterschiedlichen Siliziumanteile in den verschiedenen Teilbereichen der Beschichtung tragen maßgeblich zu Teilbereichs-spezifisch unterschiedlichen Eigenschaften bei. Insbesondere sorgt die Transportkontrollschicht dafür, dass nur langsam Material aus der Biozidschicht freigesetzt wird, während die Freisetzung eines Stoffes, insbesondere in flüssiger Form, der sich in der porösen Schicht befindet, welche als erster Teilbereich definiert ist, mit einer vergleichsweise hohen Freisetzungsrate erfolgt. Bei der porösen Schicht handelt es sich insbesondere um eine superhydrophile Schicht. Die superhydrophile Eigenschaft der Schicht bedeutet, dass ein auf die Schicht aufgetragener Wassertropfen sofort zerläuft, das heißt der Kontaktwinkel 0° beträgt.

Allgemein werden auf einem zu beschichtenden Substrat Teilbereiche verschiedener Zusammensetzung erzeugt. Hierbei wird in einem ersten Schritt ein mehrschichtiger Teilbereich, welcher aus einer Biozid-Schicht und einer diese abdeckenden Transportkontrollschicht aufgebaut wird, erzeugt. In einem weiteren Schritt wird eine Laser-Transferschicht abgeschieden, indem ein zumindest größtenteils mit Silizium beschichteter Träger vor dem Substrat platziert und anschließend in geometrisch definierter Weise per Laser bestrahlt wird.

Die poröse, mit Flüssigkeit beladbare Schicht wird mit geometrisch definierter flächiger Strukturierung auf dem aus der Biozid-Schicht und der Transportkontrollschicht aufgebauten Teilbereich erzeugt. Optional sind mehrere Teilbereiche der Beschichtung als poröse, mit Flüssigkeit beladbare, Silizium als Hauptbestandteil enthaltende Schichten ausgebildet. Diese Teilbereiche, welche als Teilbereiche erster Art definiert sind, können nebeneinander oder zumindest teilweise übereinander angeordnet sein.

Im letztgenannten Fall ist beispielsweise eine Anordnung der Schichten erster Art in einem sich kreuzenden Muster möglich. Ebenso können die porösen, zur Aufnahme von Flüssigkeit geeigneten Schichten ein Linienmuster beschreiben. In diesem Fall beträgt die Gesamtlänge des ein Linienmuster beschreibenden Teilbereichs in einer möglichen Ausgestaltung mindestens das Achtfache der Quadratwurzel der Gesamtfläche (in cm²) der beschichteten Fläche, wobei beispielsweise weniger als die Hälfte der Gesamtfläche des Substrats in Form des Linienmusters beschichtet ist.

In bevorzugter Ausgestaltung handelt es sich bei der porösen Schicht um eine Laser-Transfer-Schicht. Zum technischen Hintergrund wird beispielhaft auf die Dokumente DE 10 2018 109 337 A1 und WO 2016/055166 A2 verwiesen. Eine Laser-Transfer-Schicht wird erzeugt, indem eine transparente Folie, welche mit zu übertragendem Material, im vorliegenden Fall Silizium, beschichtet ist, auf das Substrat gelegt und anschließend gepulster Laserstrahlung ausgesetzt wird.

Die gepulste Laserstrahlung wirkt insbesondere in Form eines gerasterten Musters auf die transparente Folie ein, wobei statt einer Folie auch ein fester Gegenstand, insbesondere eine Glasplatte, zum Einsatz kommen kann. In jedem Fall ist mittels der gerasterten Lasterstrahlung eine poröse superhydrophile Schicht erzeugbar, welche in einem Muster entsprechend der Rasterung der Laserstrahlung voneinander beabstandete, näherungsweise punktförmige Regionen geringer Rauheit und Dicke aufweist. Zwischen diesen Regionen, welche auch als Laserspots bezeichnet werden, liegt ein ebenfalls der genannten porösen Schicht zuzurechnender und ebenso hauptsächlich durch auf dem Substrat niedergeschlagenes Silizium gebildeter, insgesamt netzförmiger Zwischenbereich, welcher eine vergleichsweise große Rauheit und Dicke aufweist. Insbesondere beträgt die Schichtdicke des netzförmigen Zwischenbereichs mindestens das Dreifache der Schichtdicke der Laserspots.

Verschiedene Teilbereiche der Beschichtung können sich hinsichtlich mindestens eines der Parameter mittlere Porengröße, Porosität, Hydrophilie, pH-Wert, Ladung, Polarität, Schichtdicke der porösen Schicht und mikrobielle Eigenschaften voneinander unterscheiden, wobei unterschiedliche Eigenschaften unter anderem durch eine Variation von Lasereinstellungen beim Lasertransfer erzielbar sind. Hierbei kann mindestens einer der Teilbereiche einen Gradienten mindestens eines der genannten Parameter längs seiner Oberfläche aufweisen. Beispielsweise können mehrere gleichartige, flächige Teilbereiche, welche jeweils einen Gradienten hinsichtlich eines Parameters aufweisen, direkt aneinandergesetzt sein, wobei Ränder der Teilbereiche aneinandergrenzen, welche sich hinsichtlich des betreffenden Parameters maximal voneinander unterscheiden.

Gemäß einer möglichen Weiterbildung sind innerhalb ein und desselben Teilbereichs Variationen hinsichtlich mehrerer Parameter gegeben, wobei durch diese Variationen sich voneinander unterscheidende Muster gebildet sind. Insbesondere sind hierbei durch jedes der Muster periodische, kontinuierliche oder diskontinuierliche Änderungen des betreffenden Parameters längs der Oberfläche des Teilbereichs gegeben, wobei sich die Periodenlängen der verschiedenen Muster voneinander unterscheiden.

Die sich auf der Oberfläche mehrfach, insbesondere in einem regelmäßigen Muster oder in mehreren überlagerten Mustern, ändernden Beschichtungseigenschaften haben insbesondere den Zweck, die Ausbreitung von Keimen, welche typischerweise auf ein bestimmtes Milieu angepasst sind, zu unterdrücken. Dies spielt unter anderem bei der Oberflächenbehandlung von Gegenständen, welche unter besonderen Hygienebedingungen, im Extremfall unter Reinraumbedingungen, zum Einsatz kommen, eine Rolle.

Die Beschichtung ist beispielsweise in einem medizintechnischen Implantat verwendbar. Ebenso eignet sich die Beschichtung zur Abdeckung von Wunden, wobei sich die Beschichtung in diesem Fall auf einem textilen Material befinden kann. Generell ist die Beschichtung für humanmedizinische Anwendungen ebenso geeignet wie für veterinärmedizinische Anwendungen.

Ein besonderer Vorteil der Beschichtung liegt darin, dass sich diese, sofern sie von außen zugänglich ist, praktisch beliebig oft mit Flüssigkeit nachladen lässt. Im einfachsten Fall wird lediglich mit einem getränkten Lappen über den mit flüssiger Substanz, beispielsweise einer desinfizierenden Flüssigkeit, aufzuladenden Gegenstand gewischt, wodurch dieser die Flüssigkeit in seiner porösen Struktur aufnimmt. Die Abgabe der Flüssigkeit erfolgt dagegen über einen vergleichsweise langen Zeitraum von typischerweise mehreren Tagen, wobei die Freisetzungsrate, mit der die Flüssigkeit an die Umgebung abgegeben wird, in weiten Bereichen durch die Einstellung von Parametern der Beschichtung steuerbar und auf der Gesamtoberfläche des beschichteten Produkts nicht notwendigerweise einheitlich ist.

Nachfolgend werden mehrere Ausführungsbeispiele der Erfindung anhand einer Zeichnung näher erläutert. Hierin zeigen:
- Fig. 1 bis 4: verschiedene Beschichtungsvarianten anhand schematischer Schnittdarstellungen,
- Fig. 5 bis 7: verschiedene Beschichtungsmuster in Draufsicht,
- Fig. 8: ein erstes Beispiel einer Beschichtung mit in sich uneinheitlich gestalteten Teilbereichen,
- Fig. 9 und 10: eine poröse Beschichtungsfläche mit uneinheitlichem Porendurchmesser samt zugehörigem Diagramm zur Veranschaulichung der Beladungsdichte auf der Beschichtung,
- Fig. 11 und 12: ein weiteres Beispiel einer inhomogen aufgebauten, beladbaren Beschichtungsfläche in Darstellungen analog Figuren 9 und 10,
- Fig. 13 bis 15: verschiedene beschichtete Medizinprodukte,
- Fig. 16: eine Beschichtungsfläche, innerhalb welcher sich verschiedene Parameter in Längsrichtung der Beschichtung auf verschieden Arten ändern,
- Fig. 17 bis 20: auf verschiedene Arten strukturierte, mehrfach beschichtete Flächen,
- Fig. 21 und 22: die Beschichtung eines Werkstücks mit Hilfe eines Schrumpfschlauches,
- Fig. 23 und 24: strukturiert beschichtete Flächen mit symbolisiert eingezeichneten elektrischen Ladungen,
- Fig. 25: die Ausbreitung von Stoffen längs einer in einem alternierenden Muster beschichteten Fläche.

Die folgenden Erläuterungen beziehen sich, soweit nicht anders angegeben, auf sämtliche Ausführungsbeispiele. Prinzipiell vergleichbare Komponenten oder geometrische Strukturen sind in allen Figuren mit den gleichen Bezugszeichen gekennzeichnet.

Eine insgesamt mit dem Bezugszeichen 1 gekennzeichnete Beschichtung umfasst eine Schicht 2, welche ein Biozid enthält, sowie mindestens eine poröse, superhydrophile Schicht 3. Die Beschichtung 1 befindet sich auf einem allgemein mit 7 bezeichneten Werkstück, auf dessen Oberfläche verschiedene Teilflächen 4, 5, 11 voneinander unterscheidbar sind.

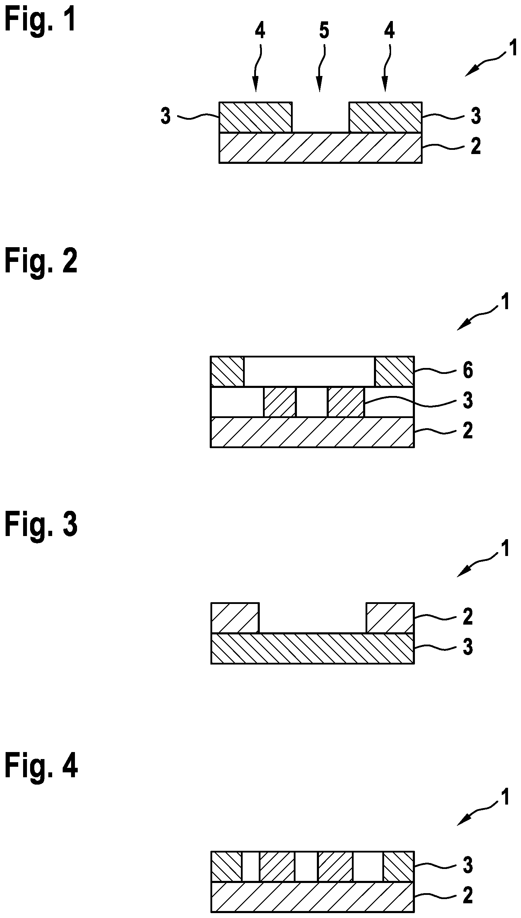

Die mindestens eine poröse, superhydrophile, siliziumbasierte Schicht 3 deckt die Biozid einhaltende Schicht 2 in den meisten Fällen zumindest teilweise ab. Die umgekehrte Schichtreihenfolge ist im Fall von Figur 3 gegeben. Die Schicht 3 umfasst in prinzipiell aus der EP 1 790 224 B1 bekannter, nicht näher dargestellter Art zusätzlich zum Biozid eine Transportkontrollschicht. Zur Abscheidung der Schicht 2 wird beispielsweise ein PVD-Verfahren genutzt. Beim Biozid, welches in der Schicht 2 enthalten ist, handelt es sich in den Ausführungsbeispielen um einen anorganischen Wirkstoff, insbesondere um Silber oder einen Silber enthaltenden Stoff. Der biozide Wirkstoff liegt in der Schicht 2 in körniger Form vor, wobei die mittlere Korngröße der Primärpartikel bevorzugt im Bereich von 5 nm bis 100 nm liegt.

Im Unterschied zur Schicht 2 ist die Schicht 3 per Lasertransfer auf das Werkstück 7 aufgebracht. In den Gestaltungen nach den Figuren 1 und 4 befindet sich die poröse superhydrophile Schicht 3 einlagig, in der Gestaltung nach Figur 2 mehrlagig auf der Schicht 2. Im letztgenannten Fall ist die zusätzliche poröse superhydrophile Schicht mit 6 bezeichnet.

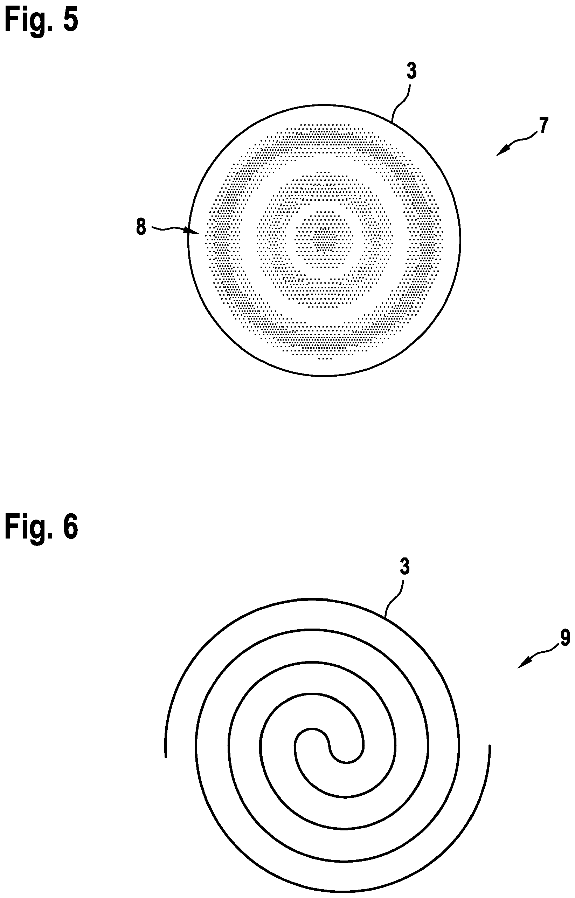

Verschiedene Möglichkeiten, mindestens eine poröse superhydrophile Schicht 3, 6 auf der Oberfläche des Werkstücks 7 zu verteilen, sind in den Figuren 5 bis 7 veranschaulicht. Gemäß Figur 5 ist durch die Schicht 3 ein Muster konzentrischer Kreise 8 auf der Substratoberfläche gebildet, wobei sich Beschichtungsparameter innerhalb der Schicht 3, wie in Figur 5 durch eine Rasterung angedeutet, vom Mittelpunkt des scheibenförmigen Werkstücks 7 aus nach außen mehrfach kontinuierlich ändern, so dass sich das Bild einer von einer Punktquelle ausgehenden Schallwelle ergibt. Jedes Maximum dieses an eine Schallwelle erinnernden Musters 8 bedeutet ein Maximum einer bestimmten Produkteigenschaft der Schicht 3. Die unterschiedlichen Rasterungen der beiden in der Anordnung nach Figur 5 erkennbaren konzentrischen, nicht scharf abgegrenzten Ringe bedeuten, dass in jedem dieser beiden ringscheibenförmigen Bereiche ein anderer Parameter der Beschichtung 1 ein Maximum aufweist. Die in geometrisch definierter Weise gestaltete Variation von Oberflächeneigenschaften auf dem Werkstück 7 spielt insbesondere hinsichtlich einer auf der Oberfläche stattfindenden Ausbreitung von Substanzen, wie anhand weiterer Ausführungsbeispiel noch erläutert werden wird, eine Rolle.

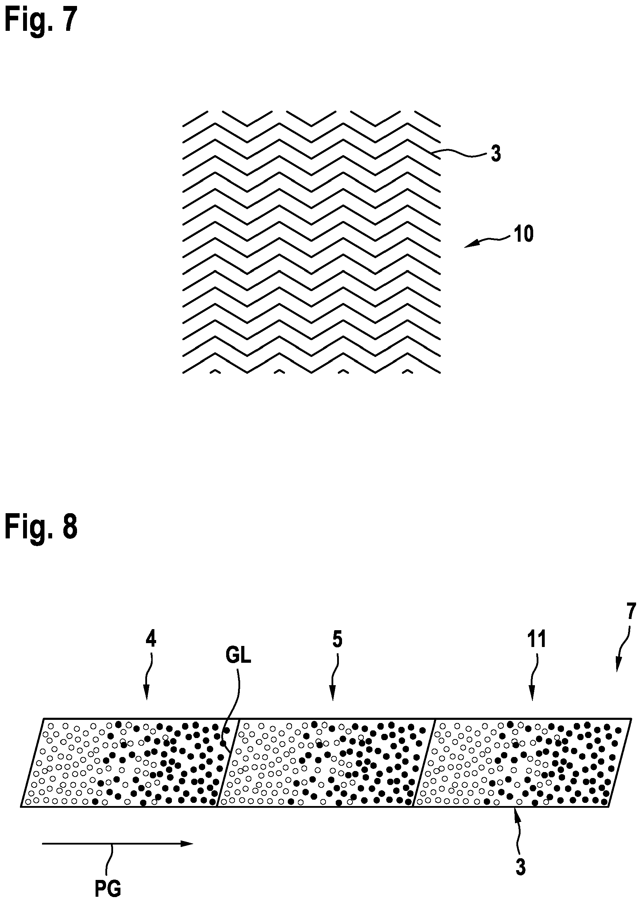

In den Ausführungsbeispielen nach den Figuren 6 und 7 ist die poröse superhydrophile Schicht 3 in Form eines Spiralmusters 9 beziehungsweise eine Zackenmusters 10 auf dem Werkstück 7 aufgetragen. Zwischenbereiche sind in beiden Fällen frei von der Schicht 3, sodass zahlreiche Übergänge zwischen verschiedenen Beschichtungsbereichen gegeben sind.

In der Ausführungsform nach Figur 8 sind verschiedene Teilflächen 4, 5, 11 in einer Reihe unmittelbar aneinandergesetzt. Innerhalb einer jeden Teilfläche 4, 5, 11 ist ein Parametergradient PG gegeben, der im vorliegenden Fall einen Ladungsgradienten bedeutet, das heißt einen kontinuierlichen Übergang zwischen "geladen" und "ungeladen" darstellt. Während sich innerhalb einer jeden Teilfläche 4, 5, 11 die Ladung nur allmählich ändert, ist an Grenzlinien GL zwischen den Teilflächen 4, 5, 11 jeweils eine sprungartige Änderung gegeben. Gerade diese sprungartigen Änderungen an den Grenzlinien GL unterdrücken unerwünschte Ausbreitungen von Mikroorganismen besonders wirksam. Anstelle eines Ladungsgradienten kann es sich bei dem Parametergradienten PG auch um einen Polaritätsgradienten, das heißt um einen Übergang von kationisch zu anionisch, handeln.

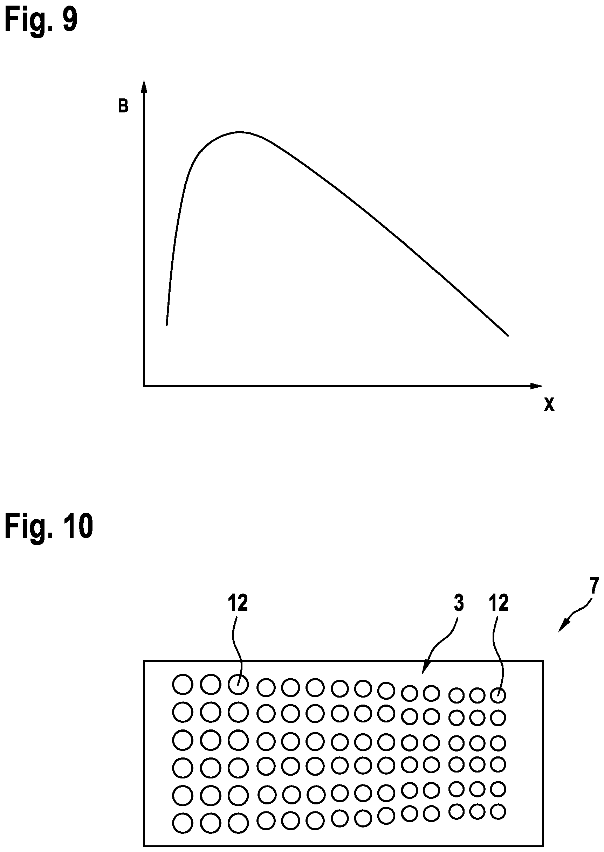

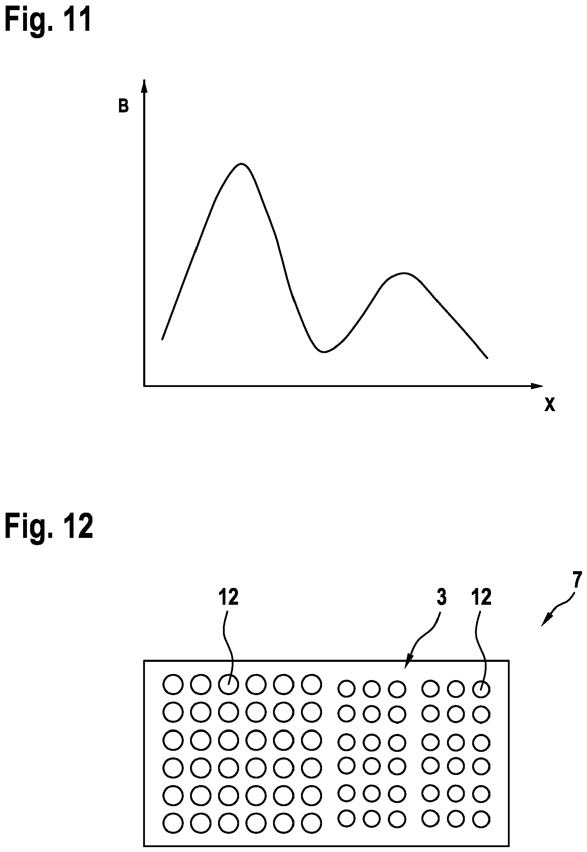

Die poröse superhydrophile Schicht 3 nach Figur 10 weist eine Vielzahl einzelner, idealisiert dargestellter Poren 12 auf, wobei sich die Porengröße in der Anordnung nach Figur 10 von links nach rechts verringert. Die gesamte Schicht 3 ist mit einer Flüssigkeit, beispielsweise einer desinfizierenden Lösung, beladbar. In Figur 9 ist die entsprechende Beladungsdichte B veranschaulicht, wobei die x-Richtung mit der Längsrichtung des Werkstücks 7 nach Figur 10 übereinstimmt. Deutlich erkennbar ist, dass im Bereich größerer Poren 12 mehr Flüssigkeit aufnehmbar ist. Dieser Zusammenhang ist grundsätzlich auch beim Ausführungsbeispiel nach den Figuren 11 und 12 gegeben, wobei in diesem Fall im Vergleich zu Figur 10 ein anderes Verteilungsmuster der Porengröße vorliegt.

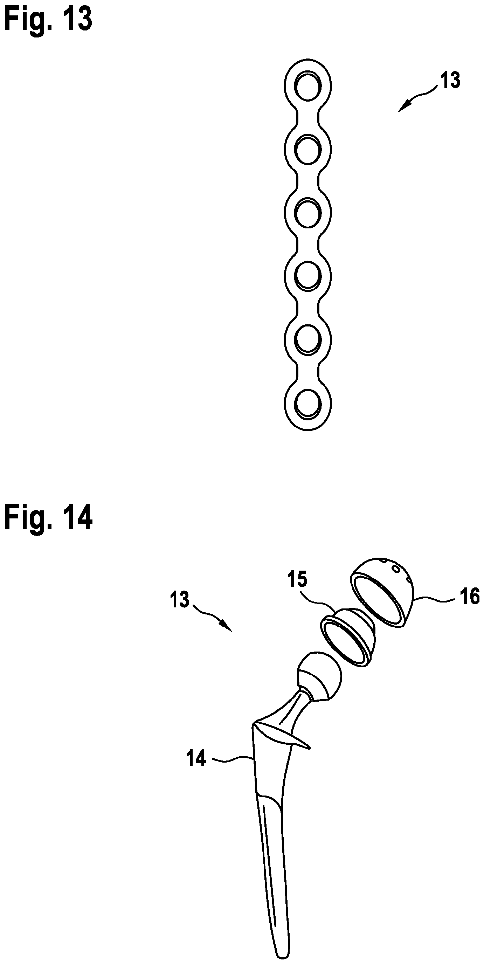

Die Figuren 13 bis 15 zeigen beispielhaft verschiedene medizinische Implantate 13 als Werkstücke 7, welche ganz oder teilweise mit der Beschichtung 1 versehen sind. Im Fall von Figur 14 sind Einzelteile des Implantats 13 mit 14, 15, 16 bezeichnet. Insgesamt handelt es sich in diesem Fall um ein künstliches Hüftgelenk. Die Beschichtung 1, welche sich zumindest teilweise auf den Einzelteilen 14, 15, 16 befindet, hat die Eigenschaft, verschiedene Substanzen, welche zum einen als Biozid in der Schicht 2 und zum anderen als Beladung der Schicht 3 vorliegen, mit unterschiedlichen Freisetzungsraten abzugeben.

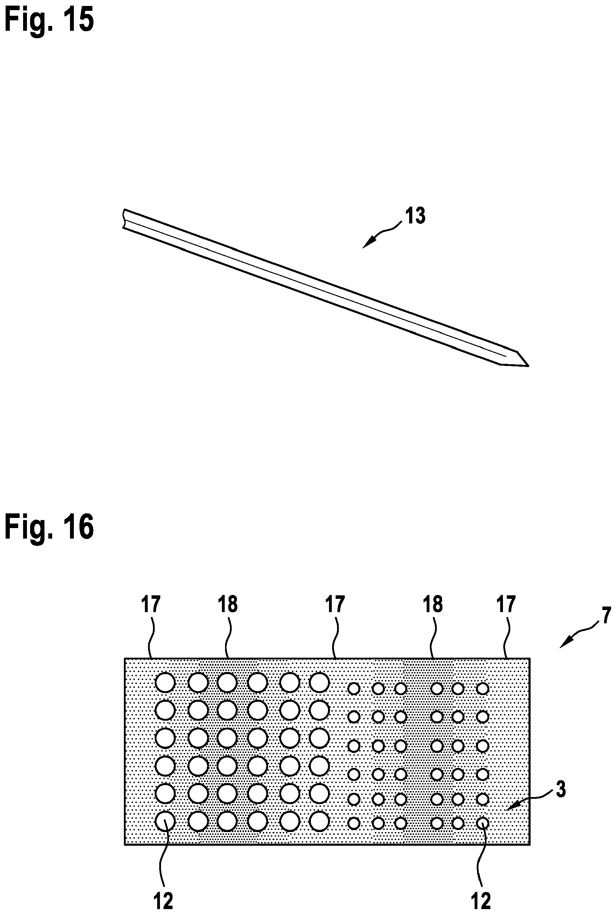

In der Ausführungsform nach Figur 16 spielen sowohl antimikrobielle als auch antibiotische und antiinflammatorische Eigenschaften der Beschichtung 1 eine Rolle. Die antimikrobiellen Eigenschaften sind hauptsächlich durch die Zusammensetzung der ein Biozid enthaltenden Schicht 2 begründet. Was die antibiotischen und antiinflammatorischen Eigenschaften betrifft, sind Subflächen 17 ersten Typs und Subflächen 18 zweiten Typs von Bedeutung, welche innerhalb der porösen Schicht 3 voneinander unterscheidbar sind. Die unmittelbar nebeneinander gesetzten Subflächen 17, 18 bilden hierbei ein Streifenmuster und bedeuten eine alternierende Polaritätsabfolge. Ferner sind innerhalb der Schicht 3 unterschiedliche Größen der Poren 12 gegeben, wie bereits anhand Figur 12 erläutert. Der regelmäßige (im Ausschnitt nach Fig. 16 lediglich einfache) Wechsel der Porengröße ist jedoch in weiteren Schritten als der Wechsel der Polarität gegeben. Somit liegen längs der Erstreckung des Werkstücks 7, das heißt in x-Richtung, an der Produktoberfläche verschiedene Parameterwechsel vor, welches jeweils mit einer bestimmten Periodenlänge erfolgen, wobei den verschiedenen Parametern verschiedene Periodenlängen zugeordnet sind.

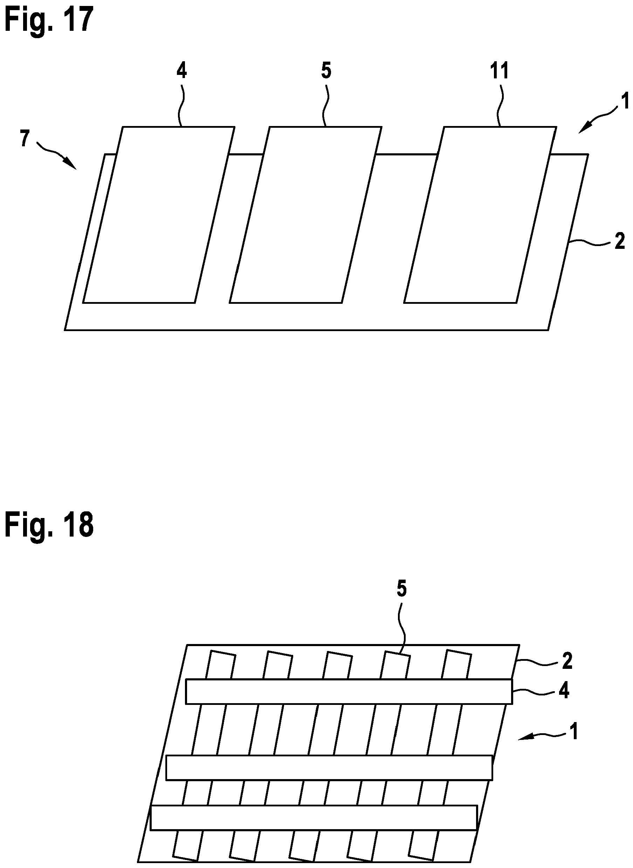

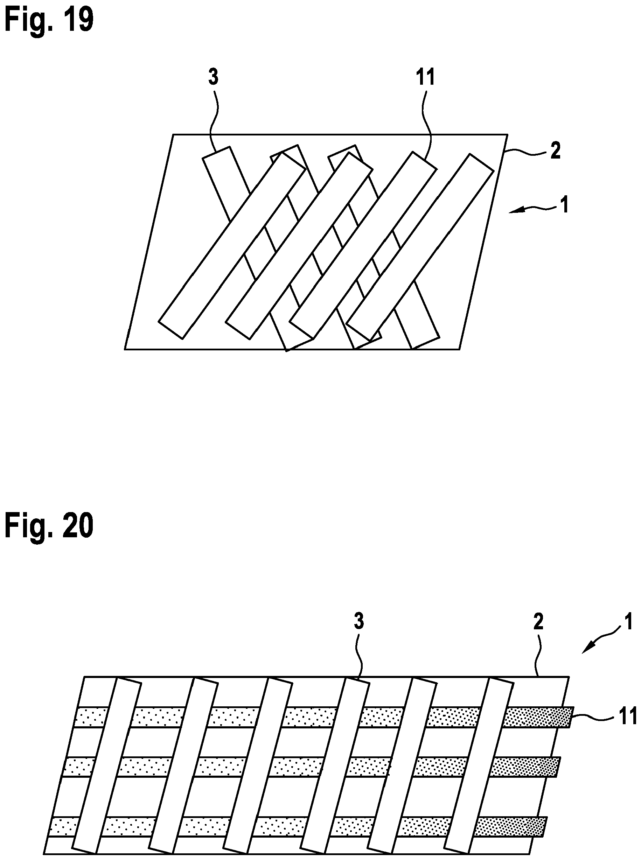

Weitere Möglichkeiten der Oberflächenstrukturierung sind in den Figuren 17 bis 20 veranschaulicht. Gemäß Figur 17 sind verschiedene Teilflächen 4, 5, 11, welche mit der porösen Schicht 3 versehen sind, mit einem Medikament beladen. Bei jeder in den Figuren 18 bis 20 dargestellten Varianten sind mehrere Lagen der Schicht 3 in sich kreuzender Weise angeordnet, wobei gemäß Figur 20 eine kontinuierliche Änderung eines Parameters längs dreier Teilflächen 11 gegeben ist.

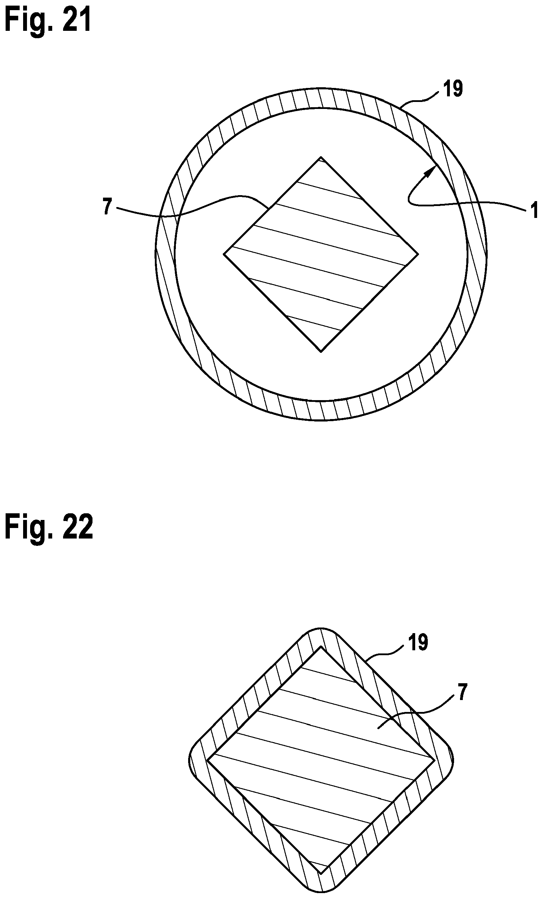

Die Figuren 21 und 22 veranschaulichen die Aufbringung der Beschichtung 1 auf der gesamten Oberfläche eines Werkstücks 7 mit Hilfe eines Schrumpfschlauches 19, auf dessen Innenseite sich die Beschichtung 1 befindet. In einem ersten Schritt wird der Schrumpfschlauch 19 über das Werkstück 7, welches unregelmäßig geformt sein kann, wie dies beispielsweise beim Einzelteil 14 der Vorrichtung nach Figur 14 der Fall ist, gezogen. Anschließend wird der Schrumpfschlauch 19, welcher aus transparentem Material sein muss, durch Erhitzen in vollflächige Anlage an die Werkstückoberfläche gebracht. Im Anschluss wird durch Bestrahlen der Beschichtung 1 mit einem nicht dargestellten Laser die Beschichtung 1 an den gewünschten Stellen, entweder partiell oder vollflächig, auf die Oberfläche des Werkstücks 7 übertragen. Auch bei diesem Vorgang ist, wie bereits erläutert, in vielfältiger Weise eine Variation der Beschichtungseigenschaften innerhalb der Beschichtungsoberfläche erzielbar.

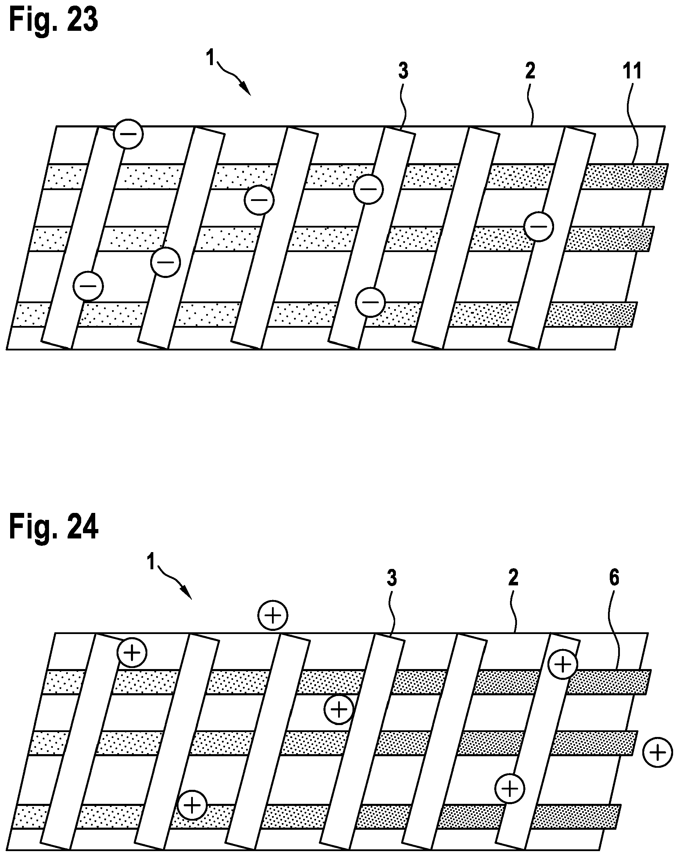

Die Ausführungsbeispiele nach den Figuren 23 und 24 unterscheiden sich vom Ausführungsbeispiel nach Figur 20 dadurch, dass definierte Polaritäten gegeben sind. Durch die unterschiedlichen Polaritäten der Schicht 3, im Fall von Fig. 23 anionisch und im Fall von Fig. 24 kationisch, kann die Freisetzung von Silberionen, welche sich in der Schicht 2 befinden, gesteuert werden.

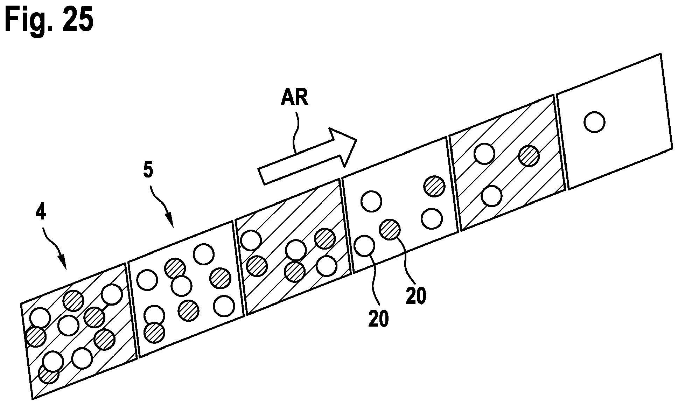

Im Fall von Figur 25 sind verschiedene Teilflächen 4, 5 alternierend aneinandergesetzt, wobei die Teilflächen 4 hydrophil und die Teilflächen 5 hydrophob sind. Symbolisch sind in Figur 25 ferner einzelne Keime 20 eingezeichnet, welche sich in Ausbreitungsrichtung AR, entsprechend der x-Richtung, ausbreiten. Wie in Figur 25 durch eine abnehmende Anzahl an Keimen 20 pro Teilfläche 4, 5 veranschaulicht ist, wirkt der ständige Wechsel zwischen verschiedenen Eigenschaften der Teilflächen 4, 5 einer Ausbreitung der Keime 20 effizient entgegen. Der prinzipiell gleiche Effekt ist erzielbar, indem die Teilflächen 4, 5 einen sich deutlich voneinander unterscheidenden pH-Wert aufweisen. Eine größere Fläche, bei der es sich beispielsweise um eine Oberfläche eines medizintechnischen Gerätes oder um einen Fußboden handelt, kann zum Beispiel schachbrettartig aus verschiedenen Teilflächen 4, 5, die sich in nicht sichtbarer Weise voneinander unterscheiden, aufgebaut sein.

### Bezugszeichenliste

- 1: Beschichtung
- 2: Biozid enthaltende Schicht, Teilbereich
- 3: poröse superhydrophile Schicht
- 4: erste Teilfläche
- 5: zweite Teilfläche
- 6: weitere poröse superhydrophile Schicht
- 7: Werkstück
- 8: Beschichtungsmuster: konzentrische Kreise
- 9: Spiralmuster
- 10: Zackenmuster
- 11: weitere Teilfläche
- 12: Pore
- 13: Werkstück
- 14: Einzelteil
- 15: Einzelteil
- 16: Einzelteil
- 17: Subfläche ersten Typs
- 18: Subfläche zweiten Typs
- 19: Schlauch
- 20: Keim

- AR: Ausbreitungsrichtung
- B: Beladungsintensität
- GL: Grenzlinie
- PG: Parametergradient

## Patentansprüche

1. Beschichtung im Medizintechnik- oder Hygienebereich, umfassend mindestens zwei verschiedene Teilbereiche (4, 5, 11), wobei mindestens einer dieser Teilbereiche (4, 5, 11) als poröse, mit Flüssigkeit beladbare, Silizium als Hauptbestandteil enthaltende Schicht (3, 6) ausgebildet und ein weiterer Teilbereich (2) mehrschichtig, nämlich aus einer Biozid-Schicht und einer diese abdeckenden Transportkontrollschicht, aufgebaut ist, **dadurch gekennzeichnet, dass** die poröse Schicht (3, 6) einen Siliziumanteil (in Gew.-%) aufweist, der mindestens das 1,4-fache und höchstens das 5-fache des Siliziumanteils der Transportkontrollschicht beträgt, und sich mindestens eine poröse Schicht (3, 6) mit geometrisch definierter flächiger Strukturierung auf dem aus der Biozid-Schicht und der Transportkontrollschicht aufgebauten Teilbereich (2) befindet.

2. Beschichtung nach Anspruch 1, **dadurch gekennzeichnet, dass** mehrere der Teilbereiche (4, 5, 11) als poröse, mit Flüssigkeit beladbare, Silizium als Hauptbestandteil enthaltende Schichten (3, 6) ausgebildet sind.

3. Beschichtung nach Anspruch 2, **dadurch gekennzeichnet, dass** mehrere der porösen Schichten (3, 6) zumindest teilweise übereinander angeordnet sind.

4. Beschichtung nach Anspruch 2 oder 3, **dadurch gekennzeichnet, dass** die porösen Schichten (3, 6) in einem sich kreuzenden Musters angeordnet sind.

5. Beschichtung nach einem der Ansprüche 1 bis 4, **dadurch gekennzeichnet, dass** der als poröse Schicht (3, 6) ausgebildete Teilbereich (4, 5, 11) ein Linienmuster beschreibt.

6. Beschichtung nach Anspruch 5, **dadurch gekennzeichnet, dass** die Liniengesamtlänge des ein Linienmuster beschreibenden Teilbereichs (4, 5, 11) mindestens das Achtfache der Quadratwurzel der Gesamtfläche (in cm²) der beschichteten Fläche beträgt, wobei weniger als die Hälfte der Gesamtfläche in Form des Linienmusters beschichtet ist.

7. Beschichtung nach einem der Ansprüche 1 bis 6, **dadurch gekennzeichnet, dass** die poröse Schicht (3, 6) als Laser-Transferschicht ausgebildet ist.

8. Beschichtung nach einem der Ansprüche 1 bis 7, **dadurch gekennzeichnet, dass** sich die verschiedenen Teilbereiche (2, 4, 5, 11) hinsichtlich mindestens eines der Parameter mittlere Porengröße, Porosität, Hydrophilie, pH-Wert, Ladung, Polarität, Schichtdicke der porösen Schicht und mikrobielle Eigenschaften voneinander unterscheiden.

9. Beschichtung nach Anspruch 8, **dadurch gekennzeichnet, dass** mindestens einer der Teilbereiche (2, 4, 5, 11) einen Gradienten (PG) mindestens eines der genannten Parameter längs seiner Oberfläche aufweist.

10. Beschichtung nach Anspruch 9, **dadurch gekennzeichnet, dass** mehrere gleichartige, flächige Teilbereiche (2, 4, 5, 11), welche jeweils einen Gradienten (PG) hinsichtlich eines Parameters aufweisen, direkt aneinandergesetzt sind, wobei Ränder der Teilbereiche aneinandergrenzen, welche sich hinsichtlich des betreffenden Parameters maximal voneinander unterscheiden.

11. Beschichtung nach einem der Ansprüche 8 bis 10, **dadurch gekennzeichnet, dass** innerhalb ein und desselben Teilbereichs (2, 4, 5, 11) Variationen hinsichtlich mehrerer Parameter gegeben sind, wobei durch diese Variationen sich voneinander unterscheidende Muster gebildet sind.

12. Beschichtung nach Anspruch 11, **dadurch gekennzeichnet, dass** durch jedes der Muster periodische, kontinuierliche oder diskontinuierliche Änderungen des betreffenden Parameters längs der Oberfläche des Teilbereichs (2, 4, 5, 11) mit unterschiedlicher Periodenlänge gegeben sind.

13. Verwendung einer Beschichtung nach Anspruch 1 in einem medizintechnischen Implantat.

14. Beschichtung nach Anspruch 1 zur Verwendung zur Abdeckung von Wunden, wobei sich die Beschichtung auf einem textilen Material befindet.

15. Verfahren zum Beschichten eines Substrats, wobei auf dem Substrat Teilbereiche (2, 4, 5, 11) verschiedener Zusammensetzung erzeugt werden, nämlich in einem ersten Schritt ein mehrschichtiger Teilbereich (2), welcher aus einer Biozid-Schicht und einer diese abdeckenden Transportkontrollschicht aufgebaut wird, und in einem weiteren Schritt eine Laser-Transferschicht (3, 6), welche abgeschieden wird, indem ein zumindest größtenteils mit Silizium beschichteter Träger vor dem Substrat platziert und anschließend in geometrisch definierter Weise per Laser bestrahlt wird.

16. Verfahren nach Anspruch 15, **dadurch gekennzeichnet, dass** als Träger ein auf seiner Innenseite beschichteter Schrumpfschlauch (19) verwendet wird, welcher über das zu beschichtende Substrat (7) gezogen und vor der Laserbestrahlung durch Hitzeeinwirkung in flächigen Kontakt mit dem Substrat (7) gebracht wird.

17. Verfahren nach Anspruch 15, **dadurch gekennzeichnet, dass** als Träger ein auf seiner Außenseite beschichteter, expandierbarer Schlauch verwendet wird, wobei dieser Schlauch in ein transparentes Rohr eingebracht und aufgeblasen wird, und wobei Beschichtungsmaterial des an der Innenwandung des Rohres anliegenden Schlauches anschließend durch von außen erfolgende Laserbestrahlung auf die Innenwandung des Rohres übertragen wird.

## Claims

1. A coating in the field of medical technology or hygiene, comprising at least two different portions (4, 5, 11), wherein at least one of these portions (4, 5, 11) is formed as a porous layer (3, 6) which can be loaded with liquid and contains silicon as the main component, and a further portion (2) is constructed from multiple layers, specifically a biocide layer and a transport control layer covering the biocide layer, **characterized in that** the porous layer (3, 6) has a silicon proportion (in wt. %) which is at least 1.4 times and at most 5 times the silicon proportion of the transport control layer, and at least one porous layer (3, 6) having a geometrically defined planar structuring is located on the portion (2) constructed from the biocide layer and the transport control layer.

2. The coating according to claim 1, **characterized in that** multiple of the portions (4, 5, 11) are formed as porous layers (3, 6) which can be loaded with liquid and contain silicon as the main component.

3. The coating according to claim 2, **characterized in that** multiple of the porous layers (3, 6) are arranged at least partially one above the other.

4. The coating according to claim 2 or 3, **characterized in that** the porous layers (3, 6) are arranged in an intersecting pattern.

5. The coating according to any one of claims 1 to 4, **characterized in that** the portion (4, 5, 11) formed as a porous layer (3, 6) describes a line pattern.

6. The coating according to claim 5, **characterized in that** the total line length of the portion (4, 5, 11) describing a line pattern is at least eight times the square root of the total area (in cm²) of the coated surface, wherein less than half of the total area is coated in the form of the line pattern.

7. The coating according to any one of claims 1 to 6, **characterized in that** the porous layer (3, 6) is formed as a laser transfer layer.

8. The coating according to any one of claims 1 to 7, **characterized in that** the various portions (2, 4, 5, 11) differ from one another with respect to at least one of the parameters average pore size, porosity, hydrophilicity, pH value, charge, polarity, layer thickness of the porous layer, and microbial properties.

9. The coating according to claim 8, **characterized in that** at least one of the portions (2, 4, 5, 11) has a gradient (PG) of at least one of the indicated parameters along its surface.

10. The coating according to claim 9, **characterized in that** multiple similar, planar portions (2, 4, 5, 11), which each have a gradient (PG) with respect to one parameter, are placed directly next to one another, wherein edges of the portions adjoin one another which differ at most from one another in terms of the respective parameter.

11. The coating according to any one of claims 8 to 10, **characterized in that** variations in terms of multiple parameters are given within one and the same portion (2, 4, 5, 11), wherein these variations form patterns which differ from one another.

12. The coating according to claim 11, **characterized in that** periodic, continuous, or discontinuous changes of the respective parameter along the surface of the portion (2, 4, 5, 11) are given by each of the patterns with different period lengths.

13. A use of a coating according to claim 1 in a medical implant.

14. The coating according to claim 1 for use in covering wounds, wherein the coating is located on a textile material.

15. A method for coating a substrate, wherein portions (2, 4, 5, 11) of different composition are created on the substrate, specifically, in a first step, a multilayer portion (2) which is constructed from a biocide layer and a transport control layer covering the biocide layer, and, in a further step, a laser transfer layer (3, 6) which is deposited by placing a carrier coated at least largely with silicon in front of the substrate and then irradiating it by laser in a geometrically defined manner.

16. The method according to claim 15, **characterized in that** a heat-shrinkable hose (19) coated on its inside is used as a carrier, which heat-shrinkable tube is pulled over the substrate (7) to be coated and is brought into planar contact with the substrate (7) by the action of heat before laser irradiation.

17. The method according to claim 15, **characterized in that** an expandable hose coated on its outer side is used as the carrier, wherein this hose is inserted into a transparent tube and inflated, and wherein coating material of the hose resting against the inner wall of the tube is then transferred to the inner wall of the tube by laser irradiation carried out from the outside.

## Revendications

1. Revêtement utilisé en technique médicale ou dans le domaine de l'hygiène, comprenant au moins deux zones partielles (4, 5, 11) différentes, au moins l'une desdites zones partielles (4, 5, 11) étant réalisée sous la forme d'une couche (3, 6) poreuse, chargeable avec un liquide, contenant du silicium en tant que composant principal et une zone partielle (2) supplémentaire étant conçue avec des couches multiples, à savoir étant construite avec une couche de biocide et une couche de contrôle du transport recouvrant celle-ci, **caractérisé en ce que** la couche (3, 6) poreuse comporte une teneur en silicium (exprimée en % en poids) qui s'élève à au moins 1,4 fois et à au plus à 5 fois la teneur en silicium de la couche de contrôle du transport et **en ce qu'**au moins une couche (3, 6) poreuse de structuration plane géométriquement définie se trouve sur la zone partielle (2) constituée par la couche de biocide et la couche de contrôle du transport.

2. Revêtement selon la revendication 1, **caractérisé en ce que** plusieurs des zones partielles (4, 5, 11) sont réalisées sous la forme de couches (3, 6) poreuses, susceptibles chargeables avec un liquide, contenant du silicium en tant que composant principal.

3. Revêtement selon la revendication 2, **caractérisé en ce que** plusieurs des couches (3, 6) poreuses sont placées au moins en superposition partielle.

4. Revêtement selon la revendication 2 ou 3, **caractérisé en ce que** les couches (3, 6) poreuses sont placées en un motif se croisant.

5. Revêtement selon l'une quelconque des revendications 1 à 4, **caractérisé en ce que** la zone partielle (4, 5, 11) réalisée sous la forme d'une couche (3, 6) poreuse décrit un motif linéaire.

6. Revêtement selon la revendication 5, **caractérisé en ce que** la longueur linéaire totale de la zone partielle (4, 5, 11) décrivant un motif linéaire s'élève au moins à 8 fois la racine carrée de la surface totale (en cm²) de la surface revêtue, moins de la moitié de la surface totale étant revêtue sous la forme du motif linéaire.

7. Revêtement selon l'une quelconque des revendications 1 à 6, **caractérisé en ce que** la couche (3, 6) poreuse est réalisée sous la forme d'une couche transfert laser.

8. Revêtement selon l'une quelconque des revendications 1 à 7, **caractérisé en ce que** les différentes zones partielles (2, 4, 5, 11) se différencient les unes des autres au niveau d'au moins l'un des paramètres taille moyenne des pores, porosité, hydrophilie, valeur pH, charge, polarité, épaisseur de couche de la couche poreuse et propriétés microbiennes.

9. Revêtement selon la revendication 8, **caractérisé en ce qu'**au moins l'une des zones partielles (2, 4, 5, 11) présente un gradient (PG) d'au moins l'un des paramètres cités le long de sa surface.

10. Revêtement selon la revendication 9, **caractérisé en ce que** plusieurs zones partielles (2, 4, 5, 11) planes de même type, lesquelles présentent chacune un gradient (PG) au niveau d'un paramètre sont directement juxtaposées, des bords des zones partielles lesquels se différencient au maximum les unes des autres au niveau du paramètre concerné étant adjacents.

11. Revêtement selon l'une quelconque des revendications 8 à 10, **caractérisé en ce qu'**à l'intérieur d'une et même zone partielle (2, 4, 5, 11) sont données des variations au niveau de plusieurs paramètres, des couches se différenciant les unes des autres étant constituées par lesdites variations.

12. Revêtement selon la revendication 11, **caractérisé en ce que** par chacun des motifs sont données des modifications périodiques continues ou discontinues du paramètre concerné le long de la surface de la zone partielle (2, 4, 5, 11) avec différente longueur de périodes.

13. Utilisation d'un revêtement selon la revendication 1 dans un implant médical.

14. Revêtement selon la revendication 1, destiné à être utilisé pour recouvrir des plaies, le revêtement se trouvant sur une matière textile.

15. Procédé, destiné à revêtir un substrat, sur le substrat étant créées des zones partielles (2, 4, 5, 11) de composition différente, à savoir dans une première étape, une zone partielle (2) à plusieurs couches, que l'on conçoit en une couche de biocide et en une couche de contrôle du transport recouvrant celle-ci et dans une étape supplémentaire, en une couche de transfert laser (3, 6), que l'on dépose en plaçant un support au moins majoritairement revêtu de silicium à l'avant du substrat et on l'irradie par la suite de manière géométriquement définie par laser.

16. Procédé selon la revendication 15, **caractérisé en ce que** l'on utilise en tant que support une gaine rétractable (19) revêtue sur sa face intérieure, que l'on tire par-dessus le substrat (7) qui doit être revêtu et avant l'irradiation au laser, que l'on amène en contact superficiel par effet thermique avec le substrat (7).

17. Procédé selon la revendication 15, **caractérisé en ce que** l'on utilise en tant que support une gaine expansible revêtue sur sa face extérieure, ladite gaine étant introduite dans une tube transparent et gonflée et de la matière de revêtement de la gaine adjacente à la paroi interne du tube étant ensuite transférée sur la paroi interne du tube par irradiation au laser, effectuée par l'extérieur.
